# EUROPEAN PATENT SPECIFICATION

(11) **EP 1 506 752 B1**
(45) Date of publication and mention of the grant of the patent: **23.01.2008**
(21) Application number: 04254795.0
(22) Date of filing: 09.08.2004
(51) Int. Cl.: A61F 2/38

(54) **Prosthesis with adjustable bearing retainer**
Prothese mit einstellbarer Lagerbefestigung
Prothèse avec support de rotation ajustable

(30) Priority: 15.08.2003 GB 0319250
(43) Date of publication of application: 16.02.2005
(73) Proprietor: Biomet UK Limited, Bridgend South Wales CF31 3XA (GB)
(72) Inventor: Parkin, Rachel, Radlett Herts WD7 8DP (GB)
(74) Representative: Giles, Ashley Simon

(56) References cited:
- EP-A- 1 186 277
- WO-A-03/063741
- US-B1- 6 296 666

## Description

This invention relates to a prosthesis with an adjustable bearing retainer. Particularly, although not exclusively, it relates to a tibial tray prosthesis having an adjustable bollard which retains a tibial bearing component.

### BACKGROUND OF THE INVENTION

Conventional knee prostheses comprise a tibial tray component which is fixed to a proximal end of a tibia, a femoral component which is fixed to a distal end of a femur and a meniscal bearing component which transfers the load between the femoral component and the tibial tray component. The meniscal component may be fixed to, or constrained by, a bollard which passes through all or part of the meniscal component and is mounted in, and projects from, a proximal end of the tibial tray component. This bollard may be integrally formed with the tibial tray prosthesis, or fixed to it in a particular position.

WO 03/063741 discloses a prosthesis having the features of the pre-characterising portion of claim 1.

### SUMMARY OF THE INVENTION

According to the present invention there is provided a prosthesis for replacement of all or part of a human or animal joint, the prosthesis comprising a mounting component for connection to a bone, a bearing component for articulation relative to a second bone, and a fixing element comprising a retainer portion which engages the bearing component and a base portion which is fixed to the mounting component, the position of the retainer portion relative to the mounting component being adjustable, characterised in that the base portion is offset from the retainer portion, so that rotation of the base portion in a bore formed in the mounting component adjusts the position of the retainer portion relative to the mounting component.

Preferably, a cross-sectional shape of the bore has rotational symmetry of at least order two.

Preferably, a cross-sectional shape of the base portion has rotational symmetry of at least order two.

Preferably, the rotational symmetry of the base portion is the same as that of the bore.

Preferably, the bore comprises an elongate recess. Preferably, the recess is symmetrical about its longitudinal axis.

Preferably, the base portion of the fixing component is elongate. Preferably, the base portion is symmetrical about its longitudinal axis.

Preferably, the base portion is closely received in the bore.

Preferably, the fixing element is fixed to the mounting component by a screw or bolt.

Preferably, an opening is provided through the fixing element to accommodate the screw or bolt.

Preferably, the fixing component can be accommodated in the bore in any one of a plurality of possible orientations. Preferably, the fixing component is reversible and can be accommodated in the bore in either of two possible orientations.

Preferably, the fixing element comprises the tibial bollard or post and the mounting component comprises the tibial tray of a knee prosthesis, and when implanted, rotation of the base portion of the bollard relative to the tibial tray displaces the retainer portion of the bollard at least in a posterior or an anterior direction.

### BRIEF DESCRIPTION OF THE DRAWINGS

For a better understanding of the present invention, and to show more clearly how it may be carried into effect, reference will now be made, by way of example, to the accompanying drawings, in which:-
Figure 1 is a perspective view of a conventional tibial component of a meniscal knee prosthesis;
Figure 2 is a cross section through the component of Figure 1;
Figure 3 is a perspective view of a tibial component of a meniscal knee prosthesis in accordance with the present invention;
Figure 4 is a cross-section through the component of Figure 3;
Figure 5 is a perspective view of a tibial bollard with an offset base portion;
Figure 6 is a plan view of the component of Figure 3 showing the bollard in a first position; and
Figure 7 is a plan view of the component of Figure 3 with the bollard in its alternative position.

### DETAILED DESCRIPTION OF A PREFERRED EMBODIMENT

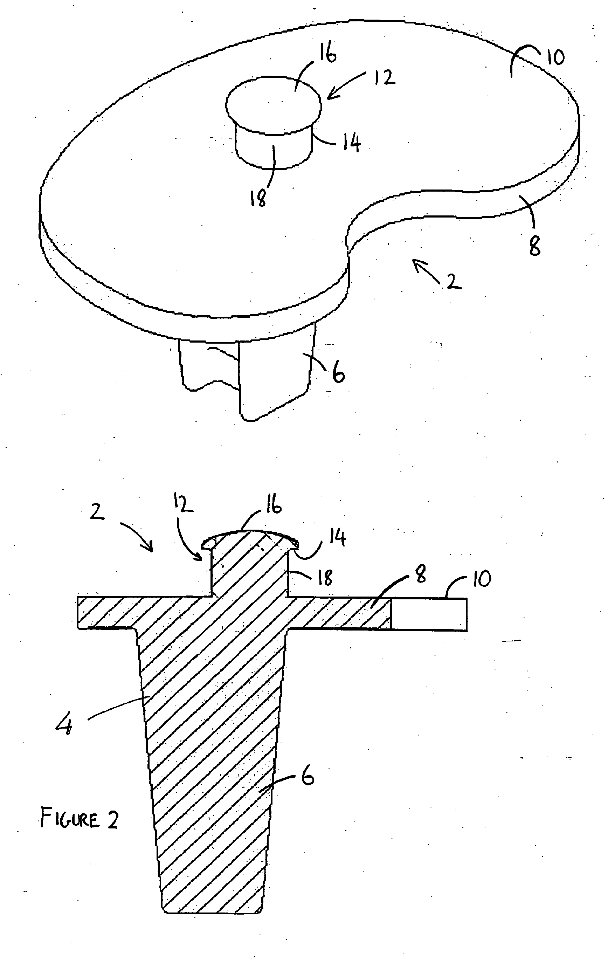

Figures 1 and 2 show a tibial component 2 of a knee prosthesis. The tibial component 2 comprises a mounting component 4 having a keel 6 which is secured in the resected end of a human tibia, and a tibial tray or platform 8 which forms a proximal bearing surface 10.

A fixing element, in the form of a tibial bollard 12, is secured centrally in the tibial platform 8, such that the bollard 12 projects proximally at right angles to the proximal bearing surface 10. An annular recess 14 is formed around the circumference of the tibial bollard 12, so that a head 16 of the bollard 12 is of larger diameter than the main body 18 of the bollard 12.

In use of the prosthesis, a meniscal bearing component (not shown) is pushed on to the bollard 12, such that the head 16 of the bollard is forced into a suitably sized opening formed through the meniscal component. When the head 16 of the bollard 12 has passed completely through the opening formed in the meniscal component, the meniscal component is retained on the bollard 12, such that it can rotate about the bollard 12 and rests against the proximal bearing surface 10 of the tibial mounting component 4.

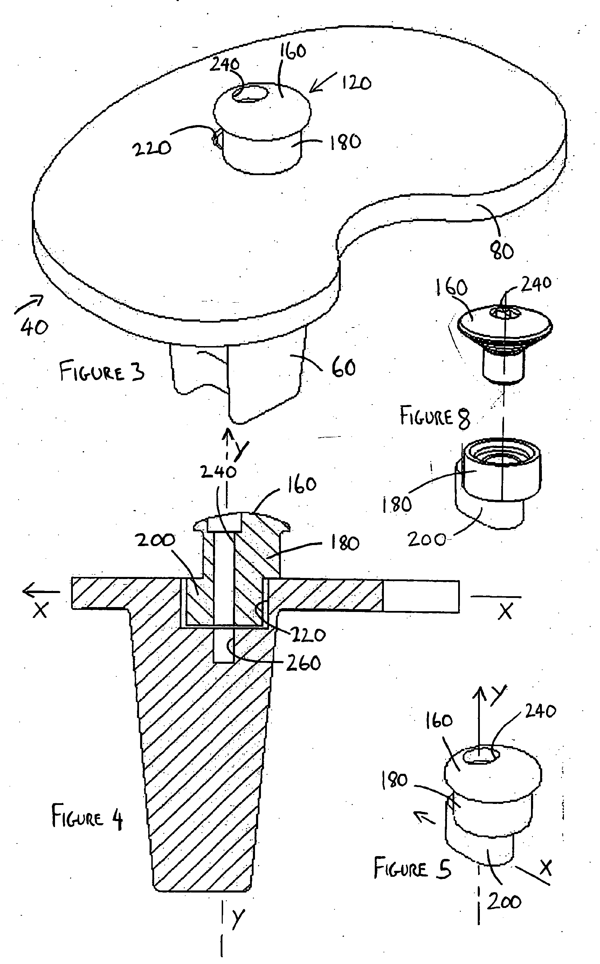

Figures 3 and 4 show a mounting component 40 in accordance with the present invention. As in the prior art arrangement described above, the mounting component 40 comprises a tibial keel 60 integrally formed with a tibial tray or platform 80. However, unlike in the prior art arrangement, a bollard 120 is releasably attached to the tibial mounting component 40, rather than being integrally formed with it. More specifically, the bollard 120 comprises a substantially cylindrical main body 180 having an oversized head 160 at its proximal end, and being integrally formed at its distal end with an offset elongate base portion 200.

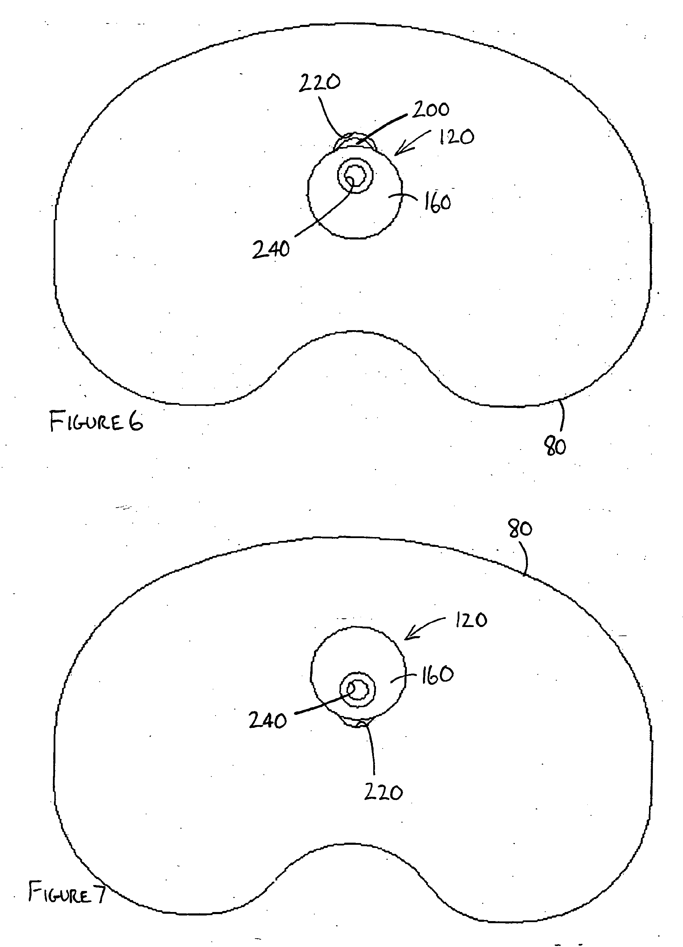

Referring to Figure 5, the elongate base portion 200 is symmetrical about a plane XY, where the X axis is an anterior/posterior axis and is parallel to a longitudinal axis of the elongate base portion 200, and the Y axis is a proximal/distal axis of the prosthesis. A corresponding elongate recess 220 is formed in the proximal end of the mounting component 40 and is adapted to receive the elongate base portion 200 closely. A counter sunk bore 240 is formed through the bollard 120 along the Y axis and passes through a centre of the elongate base portion 200. A corresponding threaded bore 260 extends along the Y axis into the keel 60 of the mounting portion 40. A screw or bolt (not shown) is passed through the counter sunk bore 240 and is screwed into the threaded bore 260 to retain the bollard 120 to the mounting portion 40.

It will be appreciated that because the base portion 200 and corresponding elongate recess 220 are symmetrical in the XY plane, with the screw or bolt removed, the bollard 120 can be withdrawn from the elongate recess 220 and rotated through a 180° before being replaced in the elongate recess 220. As a centre of the retainer portion 180 is offset along the X axis relative to the centre of the base portion 200, rotation of the base portion 200 through 180° relative to the mounting component 40 displaces the bollard 120 along the X axis relative to the tibial tray 80.

Once the mounting component 40 has been implanted, the bollard 120 can be set in one or two different positions along the X axis, such that the bollard 120 is displaced in an anterior or posterior direction relative to the tibial tray 8. Consequently, the condylar bearing surfaces formed on the meniscal component (not shown) can be displaced in an anterior or posterior direction as required by the surgeon, simply by appropriate selection of which way round the bollard 120 is assembled to the mounting component 40.

Unlike in the prior art arrangement described above, because the bollard 120 is connected to the mounting component 40 by means of a screw or bolt, it is not necessary to force the meniscal component over the bollard 120 during assembly. Instead, the meniscal component is laid on to the proximal bearing surface 100 of the mounting component 40 and the bollard is then inserted through an opening in the meniscal component and is held in place by the screw or bolt. Figures 6 and 7 show the two alternative orientations of the bollard 120 relative to the tibial platform 80. In the Figure 6 arrangement, the bollard 120 (and the meniscal component which will be connected to it) is displaced in a posterior direction, whereas in the Figure 7 embodiment, it is displaced in an anterior direction.

Although in the illustrated embodiments, a reversible bollard is illustrated which can be mounted in one or two possible orientations, it is possible, in alternative applications of the present invention for the fixing element to have a base portion which can be mounted in more than two different orientations (ie they have cross-sectional shapes which have rotational symmetry of greater than order 2). For example the base portion and corresponding recess may be triangular, so that the base portion could be mounted in any one of three possible orientations. Indeed, the base portion and corresponding recess could have any equal sided polygonal shape which is appropriate to a particular application.

Referring to Figure 8, the present invention may also be applied to a knee prosthesis having a tibial post with a removable "mushroom shaped" head 160 which may be attached to the main body 180 of the tibial post by the same screw or bolt which is used to attach the tibial post to the mounting component 40. In this embodiment, the main body 180 of the tibial post can be made a closer fit in the meniscal component because the meniscal component does not have to be forced over the head 160.

## Claims

1. A prosthesis for replacement of all or part of a human or animal joint, the prosthesis comprising a mounting component (40) for connection to a bone, a bearing component for articulation relative to a second bone, and a fixing element (120) comprising a retainer portion (180) which engages the bearing component and a base portion (200) which is connectable to the mounting component (40), the position of the retainer portion (180) relative to the mounting component (40) being adjustable, **characterised in that** the base portion (200) is offset from the retainer portion (180), so that rotation of the base portion (200) in a bore (220) formed in the mounting component (40) adjusts the position of the retainer portion (180) relative to the mounting component (40).

2. A prosthesis as claimed in claim 1, **characterised in that** a cross-sectional shape of the bore (220) has rotational symmetry of at least order two.

3. A prosthesis as claimed in claim 1 or 2, **characterised in that** a cross-sectional shape of the base portion (200) has rotational symmetry of at least order two.

4. A prosthesis as claimed in any one of claims 1 to 3, **characterised in that** the bore (220) comprises an elongate recess (220).

5. A prosthesis as claimed in any one of the preceding claims, **characterised in that** the base portion (200) of the fixing component (120) is elongate.

6. A prosthesis as claimed in any one of claims 1 to 5, **characterised in that** the base portion (200) is closely received in the bore (220).

7. A prosthesis as claimed in any one of the preceding claims, **characterised in that** the fixing element (120) is fixed to the mounting component (40) by a screw or bolt.

8. A prosthesis as claimed in claim 7, **characterised in that** an opening is provided through the fixing element to accommodate the screw or bolt.

9. A prosthesis as claimed in any one of claims 1 to 8, **characterised in that** the fixing component (120) can be accommodated in the bore (220) in any one of a plurality of possible orientations.

10. A prosthesis as claimed in claim 9, **characterised in that** the fixing component (120) is reversible and can be accommodated in the bore (220) in either of two possible orientations.

11. A prosthesis as claimed in any one of the preceding claims, **characterised in that** the fixing element (120) comprises the tibial bollard (120) and the mounting component (40) comprises the tibial tray (80) of a knee prosthesis, and when implanted, rotation of the base portion (200) of the bollard (120) relative to the tibial tray (80) displaces the retainer portion (180) of the bollard (120) at least in a posterior or an anterior direction.

12. A prosthesis as claimed in claim 11, **characterised in that** the tibial bollard (120) has a removable head (160).

## Patentansprüche

1. Prothese als vollständiger oder teilweiser Ersatz für ein Menschen- oder Tiergelenk, wobei die Prothese aufweist: eine Befestigungskomponente (40) zum Verbinden mit einem Knochen, eine Lagerkomponente zur Artikulation relativ zu einem zweiten Knochen und ein Fixierelement (120) mit einem Rückhalteteil (180), das an der Lagerkomponente und einem Basisteil (200) angreift, welches mit der Befestigungskomponente (40) verbindbar ist, wobei die Position des Rückhalteteils (180) relativ zu der Befestigungskomponente (40) einstellbar ist,
**dadurch gekennzeichnet, dass**
das Basisteil (200) von dem Rückhalteteil (180) derart versetzt angeordnet ist, dass durch Drehen des Basisteils (200) in einer in der Befestigungskomponente (40) ausgebildeten Bohrung (220) die Position des Rückhalteteils (180) relativ zu der Befestigungskomponente (40) eingestellt wird.

2. Prothese nach Anspruch 1, **dadurch gekennzeichnet, dass** die Querschnittform der Bohrung (220) eine Rotationssymmetrie mindestens der Ordnung Zwei aufweist.

3. Prothese nach Anspruch 1 oder 2, **dadurch gekennzeichnet, dass** die Querschnittform des Basisteils (200) eine Rotationssymmetrie mindestens der Ordnung Zwei aufweist.

4. Prothese nach einem der Ansprüche 1 bis 3, **dadurch gekennzeichnet, dass** die Bohrung (220) eine längliche Ausnehmung (220) aufweist.

5. Prothese nach einem der vorhergehenden Ansprüche, **dadurch gekennzeichnet, dass** das Basisteil (200) der Fixierkomponente (120) länglich ausgebildet ist.

6. Prothese nach einem der Ansprüche 1 bis 5, **dadurch gekennzeichnet, dass** das Basisteil (200) satt in der Bohrung (220) aufgenommen ist.

7. Prothese nach einem der vorhergehenden Ansprüche, **dadurch gekennzeichnet, dass** das Fixierelement (120) mittels einer Schraube oder eines Bolzens an der Befestigungskomponente (40) fixiert ist.

8. Prothese nach Anspruch 7, **dadurch gekennzeichnet, dass** eine Öffnung zum Aufnehmen der Schraube oder des Bolzens in dem Fixierelement vorgesehen ist.

9. Prothese nach einem der Ansprüche 1 bis 8, **dadurch gekennzeichnet, dass** die Fixierkomponente (120) in einer beliebigen von mehreren möglichen Ausrichtungen in der Bohrung (220) aufgenommen ist.

10. Prothese nach Anspruch 9, **dadurch gekennzeichnet, dass** die Fixierkomponente (120) umkehrbar ist und in einer von zwei möglichen Ausrichtungen in der Bohrung (220) aufgenommen ist.

11. Prothese nach einem der vorhergehenden Ansprüche, **dadurch gekennzeichnet, dass** das Fixierelement (120) den Tibianocken (120) und die Befestigungskomponente die Tibiaplattform (80) einer Knieprothese aufweisen und im implantierten Zustand durch Drehen des Basisteils (200) des Nockens (120) relativ zu der Tibiaplattform (80) der Rückhalteteil (180) des Nockens (120) zumindest in Posterieur- oder Anterieur-Richtung verschoben wird.

12. Prothese nach Anspruch 11, **dadurch gekennzeichnet, dass** der Tibianocken (120) einen abnehmbaren Kopf (160) aufweist.

## Revendications

1. Prothèse pour le remplacement de tout ou partie d'une articulation d'humain ou d'animal, la prothèse comprenant un composant de montage (40) pour le montage sur un os, un composant de support pour assurer une articulation par rapport à un deuxième os, et un élément de fixation (120) comprenant une partie de retenue (180) qui s'engage avec le composant de support et une partie de base (200) qui peut être connectée au composant de montage (40), la position de la partie de retenue (180) par rapport au composant de montage (40) étant réglable, **caractérisée en ce que** la partie de base (200) est décalée relativement à la partie de retenue (180), de sorte que la rotation de la partie de base (200) dans un trou (220) formé dans le composant de montage (40) ajuste la position de la partie de retenue (180) par rapport au composant de montage (40).

2. Prothèse selon la revendication 1, **caractérisée en ce que** la forme de la section transversale du trou (220) a une symétrie de rotation au moins d'ordre deux.

3. Prothèse selon la revendication 1 ou 2, **caractérisée en ce que** la forme de la section transversale de la partie de base (200) a une symétrie de rotation au moins d'ordre deux.

4. Prothèse selon l'une quelconque des revendications 1 à 3, **caractérisée en ce que** le trou (220) comprend un évidement allongé (220).

5. Prothèse selon l'une quelconque des revendications précédentes, **caractérisée en ce que** la partie de base (200) du composant de fixation (120) est oblongue.

6. Prothèse selon l'une quelconque des revendications 1 à 5, **caractérisée en ce que** la partie de base (200) est reçue de manière étroite dans le trou (220).

7. Prothèse selon l'une quelconque des revendications précédentes, **caractérisée en ce que** l'élément de fixation (120) est fixé au composant de montage (40) au moyen d'une vis ou d'un boulon.

8. Prothèse selon la revendication 7, **caractérisée en ce qu'**une ouverture est pratiquée dans l'élément de fixation pour recevoir la vis ou le boulon.

9. Prothèse selon l'une quelconque des revendications 1 à 8, **caractérisée en ce que** le composant de fixation (120) peut être logé dans le trou (220) dans une orientation quelconque parmi une pluralité d'orientations possibles.

10. Prothèse selon la revendication 9, **caractérisée en ce que** le composant de fixation (120) est réversible et peut être logé dans le trou (220) selon deux orientations possibles.

11. Prothèse selon l'une quelconque des revendications précédentes, **caractérisée en ce que** l'élément de fixation (120) comprend le bollard tibial (120) et le composant de montage (40) comprend le plateau tibial (80) d'une prothèse de genou, et lorsqu'elle est implantée, la rotation de la partie de base (200) du bollard (120) par rapport au plateau tibial (80) déplace la partie de retenue (180) du bollard (120) au moins dans une direction postérieure ou antérieure.

12. Prothèse selon la revendication 11, **caractérisée en ce que** le bollard tibial (120) a une tête amovible (160).
